(19)

Europäisches
Patentamt

European
Patent Office

Office européen
des brevets

(11) **EP 4 512 792 A1**

(12) **EUROPEAN PATENT APPLICATION**
published in accordance with Art. 153(4) EPC

(43) Date of publication:
**26.02.2025 Bulletin 2025/09**

(21) Application number: **23792129.1**

(22) Date of filing: **17.04.2023**

(51) International Patent Classification (IPC):
**C07C 4/22** $^{(2006.01)}$  **C07C 15/46** $^{(2006.01)}$
**C08J 11/10** $^{(2006.01)}$

(52) Cooperative Patent Classification (CPC):
**C07C 4/22; C07C 15/46; C08J 11/10;** Y02W 30/62

(86) International application number:
**PCT/KR2023/005183**

(87) International publication number:
**WO 2023/204553 (26.10.2023 Gazette 2023/43)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **18.04.2022 KR 20220047724**

(71) Applicant: **Korea Research Institute of Chemical
Technology
Daejeon 34114 (KR)**

(72) Inventors:
• **HWANG, Dong Won
Daejeon 34114 (KR)**
• **YUN, Gwang Nam
Daejeon 34114 (KR)**

• **SONG, In Hyoup
Daejeon 34114 (KR)**
• **KIM, Ji Hoon
Daejeon 34114 (KR)**
• **PARK, Jaedeuk
Daejeon 34114 (KR)**
• **MIN, Ju Won
Daejeon 34114 (KR)**
• **CHA, Seunghyeok
Daejeon 34114 (KR)**
• **EOM, In Yong
Daejeon 34114 (KR)**
• **YOO, Chang Ho
Daejeon 34114 (KR)**

(74) Representative: **Michalski Hüttermann & Partner
Patentanwälte mbB
Kaistraße 16A
40221 Düsseldorf (DE)**

(54) **METHOD FOR CONTINUOUSLY RECOVERING STYRENE MONOMER FROM WASTE
POLYSTYRENE**

(57)    The present invention relates to a method for
continuously recovering a styrene monomer from waste
polystyrene, and more specifically to a method for con-
tinuously recovering a styrene monomer from waste
polystyrene, the method comprising mixing with a potas-
sium carbonate (K2CO3) catalyst, and then continuously
depolymerizing same, thereby enabling the recovery of a
styrene monomer having high yield and high purity.

Fig. 1

EP 4 512 792 A1

**Description**

**Technical Field**

The present disclosure relates to a method of continuously recovering a styrene monomer from waste polystyrene. More specifically, the present disclosure relates to a method of continuously recovering a styrene monomer having high yield and high purity from waste polystyrene by mixing waste polystyrene with a potassium carbonate ($K_2CO_3$) catalyst and then continuously depolymerizing the mixture.

**Background Art**

With industrial development, large amounts of plastics are being used worldwide. In Korea, more than 7 million tons of general-purpose plastic products were produced last year, making Korea the fourth largest plastic producer globally. In this situation, large quantities of plastics cause many environmental problems by being discarded after use. Currently, waste plastics are mainly buried in landfills. However, waste plastics have a long biodegradation time in the soil, and landfill sites limited, leading to serious environmental problems. As a result, there is a lot of interest in developing techniques to recycle waste plastics as a resource.

Several methods have been proposed for processing waste plastics. Among the methods, recycling of the waste plastics as value-added fuel oil and raw material is considered the most desirable method than mere physically adding or processing the waste plastics in terms of the environmental issues and economic aspects. A method of recycling waste plastics is divided into a material recycling method (a method of recycling waste plastics in their original form or after processing), a thermal recycling method (such as incineration), and a chemical recycling method (a method of recovering chemicals such as resin raw materials).

The material recycling method is mainly used in recycled resin manufacturing, lightweight concrete manufacturing, and adhesive manufacturing. However, this material recycling method is a physical recycling method in which a significantly low added value is created. In addition, after multiple times of material recycling, recycling is impossible, which means generating a significant amount of waste polystyrene. Additionally, a significant amount of contaminated waste polystyrene, discharged from agricultural and fisheries markets or discharged as construction waste, is less clean than other waste polystyrene, making it difficult to use the contaminated waste polystyrene by the material recycling method.

Moreover, a significant amount of contaminated waste polystyrene has about 50 times more volume than that of other waste polystyrene, making the material recycling with the contaminated waste polystyrene difficult. In the end, the contaminated waste polystyrene has been disposed of through landfill burial or incineration. However, the incineration method causes environmental problems by generating dioxins.

Accordingly, chemical regeneration methods have been attracting attention. For example, the recovery technique of styrene, a monomer, from waste polystyrene was first attempted by Nishizaki et al. in 1997. In the conventional art, it was reported that about 50% of the monomer could be recovered from polystyrene by depolymerization at 733 K. Based on this technique, the influence of various catalysts has been examined by many researchers to increase the yield of styrene monomer, and many catalysts have been developed.

Currently, proposed methods of recovering a styrene monomer using a catalyst include a technique for recovering a styrene monomer using highly acidic metal oxides such as $Co_3O_4$, $Fe_2O_3$, $Cr_2O_3$, and CuO as a catalyst (non-patent document 0001), a technique for recovering a styrene monomer using a sulfate catalyst (Patent Documents 0001 and 0002), a dual catalyst technique for recovering a styrene monomer by bringing a metal oxide supported on silica as a main catalyst and a basic catalyst supported on alumina as a co-catalyst (patent document 0003) .

In particular, to improve the economic efficiency of the commercial process for recycling waste polystyrene, a continuous depolymerization process is more advantageous than a batch process. However, in conventional art, little research has been conducted on the continuous depolymerization process. According to the present disclosure, when waste polystyrene was continuously depolymerized over a long period using the conventional art, the activity of the depolymerization catalysts decreased as the reaction time elapsed. This illustrated that the depolymerization reaction of polystyrene was suppressed, oil production was lowered, and the yield of styrene, which is a monomer, was lowered. Additionally, it meant that by-products such as ethylbenzene, alpha-methylstyrene, benzene, and toluene was significantly increased. In the case of ethylbenzene and alpha-methylstyrene, they have almost the same boiling points as the monomer styrene. Thus, as the amount of ethylbenzene increases, styrene separating costs increases, which worsens economic efficiency.

Therefore, to commercially produce large quantities of recycled styrene from waste polystyrene, a new process technique through a continuous depolymerization process rather than through a batch depolymerization process is greatly needed for producing a styrene monomer having high yield and high purity.

[Related Art Documents]

[Patent Documents]

(Patent Document 1) Japanese Patent Application Publication No. 2001-294708 (Publication Date: 2001.10.23)
(Patent Document 2) Korean Patent Application Publication No. 2001-87093 (Publication Date: 2001.09.15)
(Patent Document 3) Korean Patent Application Publication No. 2003-0081717 (Publication Date: 2003.10.22)

[Non-Patent Document]

(Non-Patent Document 1) Ind. Eng. Res., Vol. 34, No. 12, 1995, 4519

**Disclosure**

**Technical Problem**

To address the issue, an objective of the present disclosure is to provide a method of continuously recovering a styrene monomer by suppressing the production of ethylbenzene, alpha-methylstyrene, benzene, and toluene generated in side reactions in the conventional continuous recovery process of waste polystyrene while increasing the yield of the styrene monomer.

**Technical Solution**

To achieve the objective, in one embodiment of the present disclosure, provided is a method of continuously recovering a styrene monomer from waste polystyrene by continuous depolymerization of the waste polystyrene, the method including obtaining a styrene monomer-containing product by continuously introducing waste polystyrene and a potassium carbonate ($K_2CO_3$) catalyst into a depolymerization reactor and depolymerizing the mixture, in which the yield of the styrene monomer (SM) by the depolymerization is 70% or more and satisfies one or more of Formula 1 or 2 below.

$$\text{(styrene yield)}/\text{(ethylbenzene yield)} \geq 90 \quad [\text{Formula 1}]$$

$$\text{(styrene yield)} / \text{(toluene yield + ethylbenzene yield} + \alpha\text{-methylstyrene yield)} \geq 12 \quad [\text{Formula 2}] \qquad [\text{Formula 2}]$$

In another embodiment of the present disclosure, the potassium carbonate ($K_2CO_3$) catalyst may be introduced in an amount of 0.1 to 10 parts by weight based on 100 parts by weight of waste polystyrene.

In a further embodiment of the present disclosure, the method of continuously recovering a styrene monomer from the waste polystyrene may include: (a) obtaining a styrene monomer-containing depolymerization product by continuously introducing waste polystyrene and a potassium carbonate ($K_2CO_3$) catalyst into a depolymerization reactor made of one or two or more stages and depolymerizing the mixture; (b) discharging a gaseous depolymerization product out of the depolymerization products to a depolymerization gas discharge unit using a sweeping gas, and discharging a depolymerization residue other than the gaseous depolymerization product to a residue treatment unit; and (c) obtaining a styrene monomer by collecting the discharged gaseous depolymerization product.

In a yet further embodiment of the present disclosure, the step (b) may involve supplying the sweeping gas in a counter-current to the discharge flow of the depolymerization residue to separate the styrene monomer from the depolymerization residue being discharged to a residue treatment unit and discharge the styrene monomer to the depolymerization gas discharge unit.

In a still yet further embodiment of the present disclosure, in the step (b), when the depolymerization reactor is made of two or more stages, each of the depolymerization gas discharge units, between each stage, may be connected in communication with adjacent units, allowing the gaseous depolymerization product among the depolymerization products generated in each stage of the depolymerization reactor to be discharged outside the depolymerization reactor and allowing the depolymerization residue other than the gaseous depolymerization product to be moved to the rear stage. In addition, the sweeping gas may be further supplied in a counter-current to the flow of the depolymerization residue moving from stage to stage, thereby the styrene monomer remaining in the depolymerization residue moving between each of the stages is discharged from each of the depolymerization gas discharge units.

In a still yet further embodiment of the present disclosure, the step (c) may further involve reducing the content of dimers+ in the gaseous depolymerization product discharged from the depolymerization gas discharge unit.

In a still yet further embodiment of the present disclosure, reducing the content of dimers+ may be performed in a separation column unit installed in the depolymerization gas discharge unit.

In a still yet further embodiment of the present disclosure, the sweeping gas may be heated and supplied.

In a still yet further embodiment of the present disclosure, the residence time of waste polystyrene in the depolymerization reactor may be in a range of 15 minutes to 2 hours.

In a still yet further embodiment of the present disclosure, the recovering of the potassium carbonate ($K_2CO_3$) catalyst present in the remaining residue may be further included by bringing the remaining residue into contact with water after the styrene monomer has been discharged from the residue treatment unit.

**Advantageous Effects**

A method of continuously recovering a styrene monomer from waste polystyrene according to the present disclosure induces the depolymerization of waste polystyrene under specific catalysts and conditions, thereby the production of ethylbenzene as a by-product in the styrene monomer recovery process is suppressed, and finally the styrene monomer having high yield and high purity can be recovered.

**Description of Drawings**

FIG. 1 is a process diagram of continuously recovering a styrene monomer from polystyrene using a continuous recovery device for styrene monomer according to one embodiment of the present disclosure;

FIG. 2 is a process diagram of continuously recovering a styrene monomer from polystyrene using a continuous recovery device for styrene monomer according to another embodiment of the present disclosure;

FIG. 3 is a graph measuring a product yield depending on depolymerization reaction temperature in Experimental Examples 1-1 to 1-5 according to the present disclosure;

FIG. 4 is a graph measuring a product yield depending on residence time in a depolymerization reactor in Experimental Examples 2-1 to 2-4 according to the present disclosure;

FIG. 5 is a graph measuring a product yield depending on catalyst content in Experimental Examples 3-1 to 3-4 according to the present disclosure;

FIG. 6 is a graph measuring the selective productivity of styrene monomer depending on the supply rate of sweeping gas in Experimental Examples 6-9 to 6-11 according to the present disclosure; and

FIG. 7 is a graph measuring the selective productivity of styrene monomer depending on the height of a filler material in Experimental Examples 6-5, 6-8, and 6-11 according to the present disclosure.

[Symbol Explanation]

100: Continuous recovery device for styrene monomer 110: Input device unit

111: Hopper unit 112: Open/close device unit

120: Depolymerization reactor 121: Depolymerization reactor main body unit

122: Transfer screw 123: Depolymerization gas discharge unit

124: Heat source unit 130: Residue treatment unit

131: Spray device 140: Separation column unit

**Mode for Disclosure**

Prior to a detailed description of the present disclosure, it should be noted that the present disclosure may be modified in various ways and may have several embodiments. The embodiments described below and shown in the drawings are not intended to limit the present disclosure to specific embodiments. It should be understood that all changes, equivalents, and substitutes included in the spirit and technical scope of the present disclosure are included.

When a component is referred to as being "connected" or "coupled" to another component, it may be directly connected or coupled to that other component. However, it should be understood that other components may exist in the middle. On the other hand, when it is mentioned that a component is "directly connected" or "directly coupled" to another component, it should be understood that there are no other components in between.

Terms such as "comprise", "include", or "have" used in the specification refer to the presence of features, values, steps, operations, components, parts, or combinations thereof described in the specification. It does not exclude the possibility that other features, values, steps, operations, components, parts, or combinations thereof not mentioned may exist or be added.

In addition, when describing with reference to the accompanying drawings, identical components will be assigned the same reference numerals regardless of the reference numerals, and overlapping descriptions thereof will be omitted. In describing the present disclosure, when it is determined that a detailed description of related known technologies may unnecessarily obscure the gist of the present disclosure, the detailed description will be omitted.

The present disclosure provides a method of continuously recovering a styrene monomer from waste polystyrene by continuous depolymerization of waste polystyrene, the method including obtaining a styrene monomer-containing depolymerization product by continuously introducing waste polystyrene and a potassium carbonate ($K_2CO_3$) catalyst into a depolymerization reactor and depolymerizing the mixture, in which the yield of styrene monomer (SM) by the depolymerization is 70% or more and satisfies one or more of Formula 1 or 2 below.

$$\text{(styrene yield)}/\text{(ethylbenzene yield)} \geq 90 \quad \text{[Formula 1]}$$

(styrene yield) / (toluene yield + ethylbenzene yield + $\alpha$-methylstyrene yield) $\geq$ 12 [Formula 2]         [Formula 2]

More specifically, the method of continuously recovering a styrene monomer according to one embodiment of the present disclosure may recover a styrene monomer having high yield and high purity by suppressing the production of ethylbenzene as a by-product in the styrene monomer recovery process, in which the suppression is achieved by mixing waste polystyrene with potassium carbonate ($K_2CO_3$) and continuously depolymerizing the mixture under specific conditions.

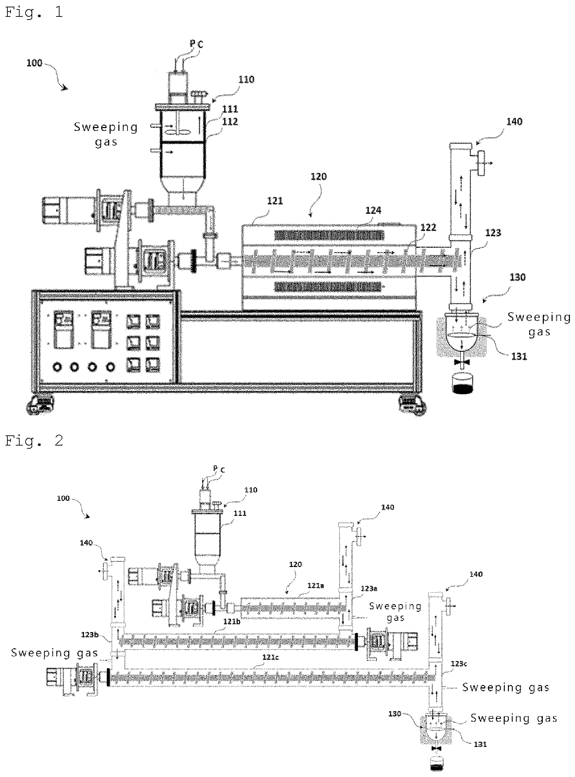

Hereinafter, the present disclosure will be described in detail with reference to the attached drawings. FIG. 1 is a process diagram of continuously recovering a styrene monomer from polystyrene using a continuous recovery device 100 for styrene monomer according to one embodiment of the present disclosure. FIG. 2 is a process diagram of continuously recovering a styrene monomer from polystyrene using a continuous recovery device 100 for styrene monomer according to another embodiment of the present disclosure.

Referring to FIGs. 1 and 2, in one embodiment of the present disclosure, the method of continuously recovering a styrene monomer from the waste polystyrene may include: (a) obtaining a styrene monomer-containing depolymerization product by continuously introducing waste polystyrene and a potassium carbonate ($K_2CO_3$) catalyst into a depolymerization reactor made of one or more stages and depolymerizing the mixture; (b) discharging a gaseous depolymerization product out of the depolymerization products to a depolymerization gas discharge unit using a sweeping gas, and discharging a depolymerization residue other than the gaseous depolymerization product to a residue treatment unit; and (c) obtaining a styrene monomer by collecting the discharged gaseous depolymerization product.

First, the method of continuously recovering a styrene monomer from waste polystyrene according to the present disclosure includes obtaining a styrene monomer-containing depolymerization product by continuously introducing waste polystyrene and a potassium carbonate ($K_2CO_3$) catalyst to a depolymerization reactor made of one or more stages and depolymerizing the mixture [Step (a)].

An input device unit 100 may be an input device unit generally applied to a depolymerization reactor. Preferably, the input device unit may be configured to include a stirring screw (not shown) in a hopper unit 111 equipped with an opening and closing device 112. Accordingly, waste polystyrene P and a catalyst S may be supplied without air (especially oxygen) when they are continuously introduced, as well as it is possible to prevent a gaseous styrene monomer-containing product generated in post-processes from leaking to the outside.

The waste polystyrene P supplied to the input device unit includes all after-use polystyrene discarded in various industrial fields. The waste polystyrene may be applied regardless of its shape and purpose. Before being introduced into the depolymerization reactor, the waste polystyrene may undergo pretreatment steps such as washing or shredding. At this point, the size of the to-be-shredded waste polystyrene may be adjusted during shredding depending on the size of a depolymerization reactor.

Meanwhile, a potassium carbonate ($K_2CO_3$) catalyst C, which is introduced into a depolymerization reactor with the waste polystyrene, is a catalyst for the depolymerization reaction of waste polystyrene. The potassium carbonate catalyst has excellent catalytic stability at high temperatures compared to other alkali or alkaline earth carbonate catalysts. The potassium carbonate catalyst facilitates recovery from depolymerization residues. When the potassium carbonate catalyst is used as a catalyst for a continuous depolymerization reaction, the yield of styrene is greatly increased, and the generation of side reaction products is greatly reduced compared to a batch reaction. Thus, the potassium carbonate catalyst may be efficiently applied to the continuous depolymerization reaction of waste polystyrene.

At this point, the potassium carbonate ($K_2CO_3$) catalyst may be introduced in an amount of 0.1 to 10 parts by weight, preferably 0.5 to 5 parts by weight, based on 100 parts by weight of waste polystyrene. When the potassium

carbonate (K$_2$CO$_3$) catalyst is present in less than 0.1 parts by weight based on 100 parts by weight of waste polystyrene, reactivity may be greatly reduced, and the amount of ethylbenzene may be greatly increased. When the amount of the potassium carbonate (K$_2$CO$_3$) catalyst exceeds 10 parts by weight based on 100 parts by weight of waste polystyrene, costs may increase without any difference in responsiveness.

Thereafter, the waste polystyrene continuously introduced into a depolymerization reactor 120 through the input device unit 110 undergoes depolymerization in the presence of the potassium carbonate (K$_2$CO$_3$) catalyst to obtain a styrene monomer-containing product.

The depolymerization reactor 120 may be used without limitation as long as the depolymerization reactor is capable of depolymerizing waste polystyrene continuously introduced. However, preferably, the depolymerization reactor may be an Auger reactor which is connected in communication with the input device unit, the Auger reactor being equipped with one or more transfer screws therein continuously transferring the waste polystyrene, which is introduced from the input device unit, from the input side to the discharge side and being made of one or two or more stages.

Specifically, as shown in FIG. 1, the depolymerization reactor according to another embodiment of the present disclosure is made of a depolymerization reactor main body unit 121 in the form of an enclosure with a space formed inside; one or more heat source units 124 provided around the depolymerization reactor main body unit; and a depolymerization gas discharge unit 123 connected to the discharge side of the depolymerization reactor main body unit to discharge depolymerization products generated in the depolymerization reactor. In addition, the depolymerization reactor may include a transfer screw 122 provided inside the depolymerization reactor main body unit to continuously transfer the introduced waste polystyrene and catalyst to generate a gaseous depolymerization product through heating obtained from the heat source unit. At this point, the heat source unit 124 may be any general heat source capable of being applied to a depolymerization reactor without limitation, and examples thereof may include burners, heaters, and heating wires.

The depolymerization reactor according to the present disclosure may be a depolymerization reactor with one main body unit as described above. However, the length of the main body unit of the depolymerization reactor may be increased as needed. In this case, considering the problem of excessive pressure caused by gas generated as waste polystyrene is heated, or the load applied to the conveying screw due to the length of the conveying screw that conveys waste polystyrene, it is preferable to have a multi-stage structure with multiple units. Accordingly, the depolymerization reactor body can be composed of two or more units, and the number of stages can be adjusted according to the depolymerization conditions.

For example, as shown in FIG. 2, a depolymerization reactor, the main body unit made of multiple stages may include a first depolymerization reactor main body unit 121a being involved in a depolymerization reaction to produce first depolymerization products with waste polystyrene and a catalyst introduced through and supplied from an input device unit 110, which is connected in communication with one end of the first depolymerization reactor main body unit; a first depolymerization gas discharge unit 123a being involved in the discharge of a first gaseous depolymerization product to the outside and the discharge of a first depolymerization residue other than the first gaseous depolymerization product to the rear stage, both of which are included in the first depolymerization products generated in the first depolymerization reactor main body unit, the discharge side of which is connected in communication with the first depolymerization gas discharge unit; a second depolymerization reactor main body unit 121b being involved in a depolymerization reaction to produce second depolymerization products with the first depolymerization residue discharged from the first depolymerization gas discharge unit 123a, one other end (other end) of which is connected in communication with the second depolymerization reactor main body unit; a second depolymerization gas discharge unit 123b being involved in the discharge of a second gaseous depolymerization product to the outside and the discharge of a second depolymerization residue other than the second gaseous depolymerization product to the rear stage, both of which are included in the second depolymerization products generated in the second depolymerization reactor main body unit, the discharge side of which is connected in communication with the second depolymerization gas discharge unit; a third depolymerization reactor main body unit 121c being involved in a depolymerization reaction to produce third depolymerization products with the second depolymerization residue, which is included in the second depolymerization products discharged from the second depolymerization reactor main body unit, the discharge side of which is connected in communication with one end (other end) of the second depolymerization gas discharge unit 123a; and a third depolymerization gas discharge unit 123c being involved in the discharge of a third gaseous depolymerization product to the outside and the discharge of a third depolymerization residue other than the third depolymerization product to the rear stage, both of which are included in the third depolymerization products generated in the third depolymerization reactor main body unit, the discharge side of which is connected in communication with the third depolymerization gas discharge unit.

In the multi-stage depolymerization reactor configured as described above, the waste polystyrene introduced from the input device unit is transferred in a zig-zag flow and is depolymerized by heating in the heat source unit to produce the depolymerization products. The generated depolymerization products are separated into a gaseous depolymerization product (dotted arrow) and a depolymerization residue other than the gaseous depolymerization product (solid arrow) depending on the boiling point. The separated gaseous depolymerization product (dotted arrow) is discharged to the outside or to a separation column unit 140, which will be described later. The depolymerization residue (solid arrow) is discharged to a residue treatment unit.

Here, the depolymerization reaction temperature, residence time, and rotation of the transfer screw may be controlled in the depolymerization reactor to adjust the yield of the styrene monomer produced. In the multi-stage depolymerization reactor, the depolymerization reaction temperature, residence time, and stirring speed of the transfer screw in each depolymerization reactor main body may also independently be controlled to adjust the yield of styrene monomer.

At this point, the depolymerization reaction temperature in the depolymerization reactor may be in a range of 420°C to 550°C, preferably 450°C to 500°C, more preferably 450°C to 475°C. When the depolymerization reaction temperature is less than 420°C, the amount of residual oil and high-boiling dimers and trimers produced increases due to a decrease in reactivity, and the amount of styrene monomer produced may be significantly reduced. When the depolymerization reaction temperature exceeds 550°C, the amount of benzene and toluene may greatly increase due to the cracking reaction, which may significantly reduce the amount of styrene monomer produced, and dimers and trimers which have a high boiling point may be mixed into the products.

Meanwhile, the residence time of polystyrene in the depolymerization reactor may be in a range of 15 minutes to 2 hours, preferably in a range of 20 minutes to 1 hour. When the residence time is less than 15 minutes or more than 2 hours, the yield of the styrene monomer may be lowered, and the amount of the styrene monomer produced per hour may be significantly lowered.

The products obtained from the depolymerization reaction include a styrene monomer (SM), which is the final target component, as well as ethylbenzene (EB), toluene, cumene, alpha methylstyrene, substances with a high boiling point such as dimers+, and depolymerization residues. These depolymerization residues not only interfere with the depolymerization reaction but also reduce the yield of styrene monomer. The dimers+ refer to compounds containing two or more benzene rings. The dimers+ may include 1,3-diphenylpropane, 4-diphenyl-1-butane, 1-phenylnaphthalene, 2-benzylnaphthalene, m-terphenyl, 1,3,5-triphenylcyclohexane, and 1,3,5-triphenylbenzene.

Therefore, in the method of continuously recovering a styrene monomer according to the present disclosure, a potassium carbonate catalyst is used for depolymerization under specific conditions, thereby it is possible to continuously obtain 70% or more yield of styrene monomer, the final target component, as well as ethylbenzene (EB), toluene, cumene, and alpha methylstyrene may each be recovered at a yield of 3.5% or less.

In particular, in the method of continuously recovering a styrene monomer according to the present disclosure, the yield of styrene monomer may be 70% or more and satisfy one or more of Formula 1 or 2 below. Preferably, the yield of the styrene monomer may satisfy one or more of Formulas 1 to 3, and even more preferably may satisfy two or more of Formulas 1 to 3.

$$\text{(styrene yield)}/\text{(ethylbenzene yield)} \geq 90 \quad \text{[Formula 1]}$$

$$\text{(styrene yield)} / \text{(toluene yield + ethylbenzene yield} + \alpha\text{-methylstyrene yield)} \geq 12 \text{ [Formula 2]} \quad \text{[Formula 2]}$$

$$\text{(styrene yield)}/\text{(dimer+ yield)} \geq 15 \quad \text{[Formula 3]}$$

That is, by the method of recovering styrene monomer according to the present disclosure, a later process described later to recover the styrene monomer, the final target component, may easily be performed by suppressing the production of ethylbenzene, alpha-methylstyrene, and toluene, and especially ethylbenzene, which are generated in side reactions in the conventional styrene monomer recovery process. Through the later process, it is possible to further improve the yield and purity of the final styrene monomer.

As mentioned above, among depolymerization products generated in the depolymerization reactor, a gaseous depolymerization product (dotted arrow) is discharged to the depolymerization gas discharge unit using a sweeping gas, and a depolymerization residue is discharged to the residue treatment unit [step (b)].

At this point, to allow the gaseous depolymerization product generated by depolymerization to be easily discharged from the depolymerization reactor and move to the outside or the separation column unit 140, the sweeping gas may be supplied from the input side to the discharge side of the depolymerization reactor. Any method of supplying the sweeping gas may be applied without limitation as long as the sweeping gas may be supplied to the depolymerization reactor by the method. For example, the sweeping gas may be supplied to the input device unit when waste polystyrene is introduced into the input device unit of the depolymerization reactor.

The sweeping gas may be used without limitation as long as the sweeping gas is a non-reactive gas such as nitrogen, argon, and helium, and the sweeping gas may be supplied after being heated. The temperature of the sweeping gas supplied may be in a range of 20°C to 500°C. The temperature may be preferably raised to 200°C to 400°C in terms of depolymerization reaction efficiency.

Meanwhile, the depolymerization residue remaining after having been depolymerized in the depolymerization reactor is continuously introduced into the residue treatment unit 130 and then heated, thereby the styrene monomer remaining in the depolymerization residue may be recovered, and the remaining residue may be discharged to the outside or moved back to the polystyrene inlet 110. The remaining catalyst may be recovered using water from the remaining residue discharged to the outside.

One end of the residue treatment unit 130 is connected in communication with the depolymerization gas discharge unit 123 of the depolymerization reactor. Thus, the liquid depolymerization residue remaining after having been depolymerized in the depolymerization reactor is supplied to the residue treatment unit. The supplied depolymerization residue is heated to recover the gaseous styrene monomer remaining in the depolymerization residue and discharge the gaseous styrene monomer to the outside or the separation column unit 140 through the depolymerization gas discharge unit.

At this point, the temperature of the residue treatment unit may be in a range of 100°C to 300°C in terms of improving the yield and selectivity of styrene monomer.

Meanwhile, by the method of continuously recovering a styrene monomer from waste polystyrene according to a further embodiment of the present disclosure, a sweeping gas is sprayed into the discharge flow of the depolymerization residue in a counter-current to the discharge flow of the depolymerization residue discharged from the depolymerization gas discharge unit of the depolymerization reactor to increase the recovery and selectivity of styrene monomer. Through this, the styrene monomer remaining in the depolymerization residue is separated from the depolymerization residue and is moved back to the depolymerization gas discharge unit.

The spraying of the sweeping gas onto the depolymerization residue is performed by using a sweeping gas spray device 131 in a counter-current to the flow of the depolymerization residue being supplied to the residue treatment unit, thereby helping separate the styrene monomer from the depolymerization residue and move the styrene monomer to the depolymerization gas discharge unit. Accordingly, polymerization caused by the stagnation of styrene monomer at a rear stage of the depolymerization reactor is prevented. As a result, the recovery rate of styrene monomer may be increased. The sweeping gas spray device may be used without limitation as long as the sweeping gas spray device is a spray device capable of spraying gas.

At this point, the sweeping gas may be a non-reactive gas such as nitrogen, argon, and helium, or a light hydrocarbon such as methane and ethane, and may be supplied by heating.

Additionally, the sweeping gas sprayed in a counter-current to the residue flow may be supplied at a rate in a range of 0.01 cm/min to 20 cm/min, preferably in a range of 0.5 cm/min to 10 cm/min. When the supply rate of the sweeping gas is less than 0.01 cm/min, the flow in a counter-current is lowered, so the styrene monomer produced may stagnate, and polymerization may occur. In addition, the separation ability of styrene monomer from the depolymerization residue may also be lowered, which may reduce the yield of styrene monomer. When the supply rate of the sweeping gas exceeds 20 cm/min, products with a relatively high boiling point such as the dimers+ remaining in the depolymerization residue pass through the separation column unit 140 together with the styrene monomer and move to the depolymerization gas discharge unit, so the selectivity of the styrene monomer may be lowered.

In addition, in the method of continuously recovering a styrene monomer from waste polystyrene according to a yet further embodiment of the present disclosure, the depolymerization reactor may be a multi-stage depolymerization reactor made of two or more stages. In that case, as mentioned above, a plurality of depolymerization gas discharge units 123a and 123b are provided connected to one side of one of the depolymerization reactor main body units. A residue treatment unit 130 is connected only to the third depolymerization gas discharge unit 123c connected in communication with the final third depolymerization reactor main body unit. In this situation, by supplying a sweeping gas in the counter-current to the flow of each of the depolymerization residues supplying to one of the depolymerization reactor main body units at the rear stage through each of the plurality of depolymerization gas discharge units, the styrene monomer remaining in each of the depolymerization residues being supplied to one of the depolymerization reactor main body units at the rear stage may be recovered without disposal. After the styrene monomer has been recovered from the preceding depolymerization residue, the final remaining depolymerization residue is discharged to the residue treatment unit 130.

Meanwhile, the method of continuously recovering a styrene monomer from waste polystyrene according to a still yet further embodiment of the present disclosure may include recovering a potassium carbonate ($K_2CO_3$) catalyst from the remaining residue by bringing the remaining residue into contact with water after the styrene monomer has been recovered.

The potassium carbonate ($K_2CO_3$) catalyst used in the depolymerization reaction is continuously discharged from the residue treatment unit 130 with the remaining residue. At this point, the potassium carbonate catalyst included in the discharged remaining residue is water-soluble, unlike the components of the remaining residue. Thus, by bringing the potassium carbonate catalyst-including remaining residue into contact with water, only the potassium carbonate catalyst may be dissolved to be separated and recovered from the remaining residue, and the separated and recovered potassium carbonate catalyst may be recycled for the depolymerization reaction through water removal.

Thereafter, the styrene monomer-containing gaseous depolymerization product that is moved or discharged from the depolymerization gas discharge unit is collected to obtain a styrene monomer [step (c)].

At this point, the present disclosure may further include reducing the content of dimers+ in the gaseous depolymerization product discharged from the depolymerization gas discharge unit. The reducing of the content of dimers+ may be performed using a separation column unit 140 connected in communication with one side of the depolymerization gas discharge unit.

In a still yet further embodiment of the present disclosure, the separation column unit 140 is connected by having its one side in communication with the depolymerization gas discharge unit 123 of the depolymerization reactor. Thus, the gaseous depolymerization product obtained in the depolymerization reactor 120 and the styrene monomer recovered in the residue treatment unit 130 may be supplied to the separation column unit and liquefied. This may lead to removing dimers+ with a low boiling point from the gaseous depolymerization product, which is supplied from the depolymerization gas discharge unit, thereby the styrene monomer may be obtained.

Meanwhile, in a still yet further embodiment of the present disclosure, when a separation column unit 140 is connected to a multi-stage depolymerization reactor, a plurality of the separation column unit may be connected to one side of each of the depolymerization gas discharge units in the depolymerization reactor. For example, as shown in FIG. 2, each of the separation column units 140 is connected to each of the depolymerization gas discharge units in the multi-stage depolymerization reactor, thereby a gaseous depolymerization product discharged from each of the depolymerization gas discharge units may be supplied to each of the separation column unit. The depolymerization gas discharge unit at the last stage is connected to the residue treatment unit, so with the styrene monomer recovered from the residue treatment unit, the gaseous depolymerization product discharged from the depolymerization gas discharge unit may be liquefied.

At this point, the separation column unit 140 may be a separation column unit filled with a filler material or a separation column unit not filled with a filler material. The gaseous styrene monomer recovered from the residue treatment unit and the gaseous depolymerization product obtained from the depolymerization reactor may be supplied to undergo a phase transition to a liquid state as they pass through the separation column unit, thereby substances with a high boiling point, such as dimers+, may be further removed. As the filler material, borosilicate, porcelain, and stainless steel may be used.

At this point, as the volume ratio (filling ratio) of the separation column unit filled with the filler material increases, the purity of styrene monomer may be further improved whereas the yield of styrene monomer decreases. Based on this, the yield and purity of styrene monomer may be appropriately adjusted.

Next, the styrene monomer that has passed through the separation column unit may be recovered by collecting and cooling. At this point, cooling is an indirect cooling method of cooling a collected product by compressing and evaporating a refrigerant on the basis of the evaporation heat of the refrigerant. However, any cooling methods applied in the industry, such as direct cooling, may be used without limitation.

Hereinafter, the present disclosure will be described in more detail through specific examples. The following experimental examples are merely examples to aid understanding of the present disclosure, and the scope of the present disclosure is not limited thereto.

**[Experimental Example 1: Measurement of yield and selectivity of depolymerization product depending on depolymerization reaction temperature]**

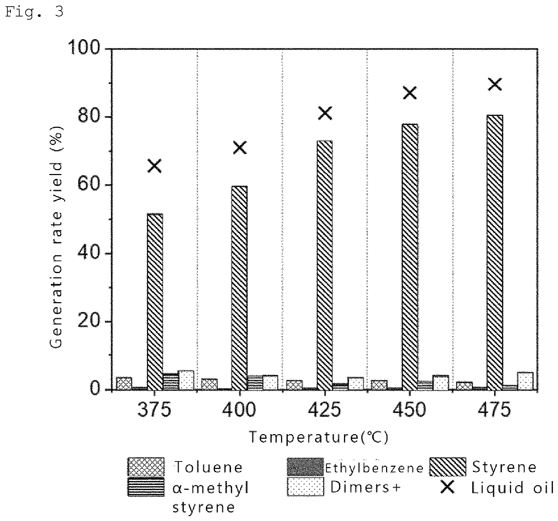

1 kg of shredded waste polystyrene (PS) with an average particle size of 0.5 cm was mixed with 50 g (5 wt%) of potassium carbonate ($K_2CO_3$) (Sigma-Aldrich, ACS reagent > 99%). Next, the mixture was introduced into an Auger reactor shown in FIG. 1 through an input device unit with nitrogen gas supplied at a rate of 5 cm/min. The mixture was subjected to stirring for 22 minutes at a rate of 2.5 rpm under the reaction conditions listed in Table 1 to perform a continuous depolymerization reaction. At this point, a sweeping gas spray nozzle was installed in the residue treatment unit, and nitrogen gas (200°C), which was a sweeping gas, was sprayed at a supply rate of 0.5 cm/min in the counter-current to the inflow of the depolymerization residue. The depolymerization product was liquefied without installing a separation column unit. Then, the liquefied product was subjected to measurement using a GC/FID (Younglin Instruments) equipped with a capillary column (HP-5, 30 m × 0.32 mm × 1.0 μm, Crosslinked 5 % PH ME Siloxane). The results are shown in Tables 1 and 2 and FIG. 3. $\alpha_1$, $\alpha_2$, and $\alpha_3$ in Table 1 below were calculated using Formulas 1 to 3 below. At this point, SM was a styrene monomer, EB was ethylbenzene, and $\alpha$-MS was alpha-methylstyrene.

$$\alpha_1= \text{(styrene yield)/(ethylbenzene yield)} \quad \text{[Formula 1]}$$

$$\alpha_2= \text{(styrene yield)/ (toluene yield + ethylbenzene yield + } \alpha\text{-methylstyrene yield)} \text{ [Formula 2]} \quad \text{[Formula 2]}$$

$$\alpha_3 = \text{(styrene yield)/(dimer+ yield)} \quad \text{[Formula 3]}$$

[Table 1]

| Division | Depolymerization reaction temperature (° C) | Yield (%) | | | | | | $\alpha_1$ | $\alpha_2$ | $\alpha_3$ |
|---|---|---|---|---|---|---|---|---|---|---|
| | | Liquid oil | Dimers+ | Toluene | EB | SM | $\alpha$-MS | | | |
| Experimental Example 1-1 | 375 | 65.7 | 5.5 | 3.5 | 0.6 | 51.5 | 4.6 | 85.8 | 5.9 | 9.4 |
| Experimental Example 1-2 | 400 | 71.0 | 4.2 | 3.1 | 0.2 | 59.6 | 3.9 | 298.0 | 8.3 | 14.2 |
| Experimental Example 1-3 | 425 | 81.1 | 3.5 | 2.6 | 0.4 | 72.8 | 1.8 | 182.0 | 15.2 | 20.8 |
| Experimental Example 1-4 | 450 | 87.1 | 4.1 | 2.6 | 0.5 | 77.8 | 2.1 | 155.6 | 15.0 | 19.0 |
| Experimental Example 1-5 | 475 | 89.6 | 5.1 | 2.2 | 0.6 | 80.5 | 1.2 | 134.2 | 20.1 | 15.8 |
| SM: styrene monomer, EB: Ethylbenzene, $\alpha$-MS: alphamethylstyrene | | | | | | | | | | |

[Table 2]

| Division | Selectivity (%) | | | | |
|---|---|---|---|---|---|
| | Dimers+ | Toluene | EB | SM | $\alpha$-MS |
| Experimental Example 1-1 | 8.4 | 5.3 | 0.9 | 78.4 | 7.0 |
| Experimental Example 1-2 | 5.9 | 4.4 | 0.3 | 83.9 | 5.5 |
| Experimental Example 1-3 | 4.3 | 3.2 | 0.5 | 89.8 | 2.2 |
| Experimental Example 1-4 | 4.7 | 3.0 | 0.6 | 89.3 | 2.4 |
| Experimental Example 1-5 | 5.7 | 2.5 | 0.7 | 89.8 | 1.3 |
| SM: styrene monomer, EB: Ethylbenzene, $\alpha$-MS: alphamethylstyrene | | | | | |

As shown in Tables 1 and 2 and FIG. 3 below, when the depolymerization reaction temperature was 425°C or higher as in Experimental Examples 1-3 to 1-5, the yield and selectivity of styrene monomer were found to be high compared to those in Experimental Examples 1-1 and 1-2 where the depolymerization reaction temperature was 400°C or lower. In particular, $\alpha_1$, $\alpha_2$, and $\alpha_3$ were 90 or more, 12 or more, and 15 or more, respectively, in Experimental Examples 1-3 to 1-5, so it was confirmed that the amount of by-products was small.

**[Experimental Example 2: Measurement of yield of depolymerization product depending on residence time]**

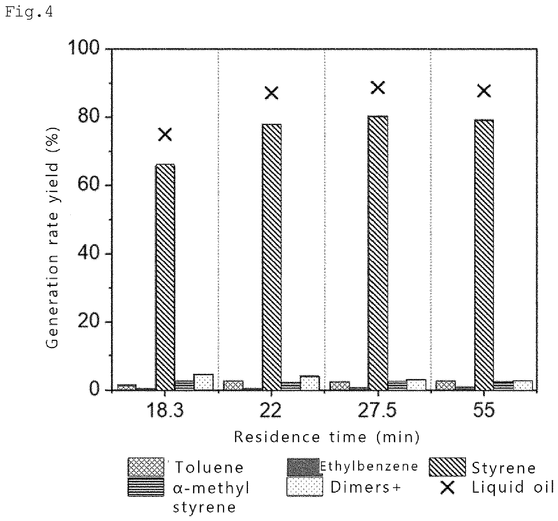

1 kg of shredded waste polystyrene (PS) with an average particle size of 0.5 cm was mixed with 50 g (5 wt%) of potassium carbonate ($K_2CO_3$) (Sigma-Aldrich, ACS reagent > 99%). Next, the mixture was introduced into an Auger reactor shown in FIG. 1 through an input device unit with nitrogen gas supplied at a rate of 5 cm/min. The mixture was subjected to continuous depolymerization at a temperature of 450°C under the reaction conditions listed in Table 3. At this point, a sweeping gas spray nozzle was installed in the residue treatment unit, and nitrogen gas (200°C), which was a sweeping gas, was sprayed at a supply rate of 0.5 cm/min in the counter-current to the inflow of the depolymerization residue. The depolymerization product was liquefied without installing a separation column unit. Then, the liquefied product was subjected to measurement using a GC/FID (Younglin Instruments) equipped with a capillary column (HP-5, 30 m × 0.32 mm × 1.0 μm, Crosslinked 5 % PH ME Siloxane). The results are shown in Table 3 and FIG. 4 below. $\alpha_1$, $\alpha_2$, and $\alpha_3$ in Table 3 below were calculated using Formulas 1 to 3 above.

[Table 3]

| Division | Depolymerization reactor Residence time (min) | Yield (%) | | | | | | $\alpha_1$ | $\alpha_2$ | $\alpha_3$ |
|---|---|---|---|---|---|---|---|---|---|---|
| | | Liquid oil | Dimers+ | Toluene | EB | SM | $\alpha$-MS | | | |
| Experimental Example 2-1 | 18.3 | 74.9 | 4.6 | 1.5 | 0.4 | 65.8 | 2.6 | 164.5 | 14.6 | 14.3 |
| Experimental Example 2-2 | 22 | 87.1 | 4.1 | 2.6 | 0.5 | 77.8 | 2.1 | 155.6 | 15.0 | 19.0 |

| Division | | | | | | | | | | |
|---|---|---|---|---|---|---|---|---|---|---|
| Experimental Example 2-3 | 27.5 | 88.7 | 3.1 | 2.4 | 0.6 | 80.3 | 2.3 | 133.8 | 15.2 | 25.9 |
| Experimental Example 2-4 | 55 | 87.7 | 2.7 | 2.6 | 0.8 | 79.1 | 2.5 | 98.9 | 13.4 | 29.3 |
| SM: styrene monomer, EB: Ethylbenzene, $\alpha$-MS: alphamethylstyrene | | | | | | | | | | |

As shown in Table 3 and FIG. 4, in Experimental Examples 2-2 to 2-4, where the residence time was longer than that of Experimental Example 2-1, the yield of styrene monomer was found to be high. From this, it was assumed that residence time in the depolymerization reactor was shortened due to the high rotation speed of Experimental Example 2-1, which caused non-sufficient depolymerization, leading to a lowered yield of styrene monomer, and $\alpha_3$ was lowered due to the increase in unreacted dimer+.

[Experimental Example 3: Measurement of yield of depolymerization product depending on catalyst content for depolymerization reaction]

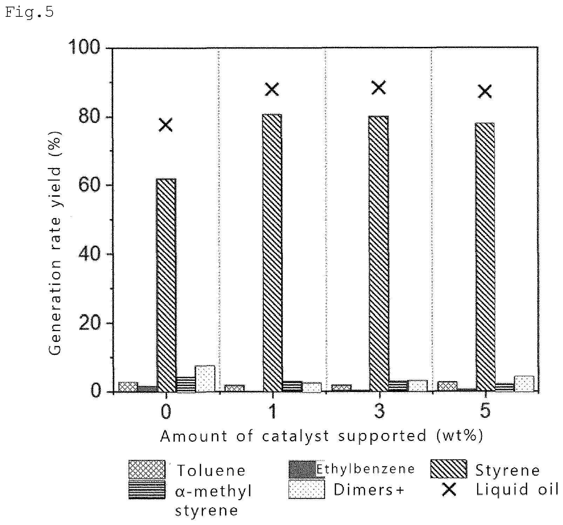

1 kg of waste polystyrene (PS) shredded to an average particle size of 0.5 cm and potassium carbonate ($K_2CO_3$) (Sigma-Aldrich, ACS reagent > 99%) were mixed under the conditions shown in Table 4 below. Next, the mixture was introduced into an Auger reactor shown in FIG. 1 through an input device unit with nitrogen gas supplied at a rate of 5 cm/min. The mixture was subjected to stirring at a temperature of 450°C for 22 minutes (a stirring rate of 2.5 rpm) under the reaction conditions listed in Table 4 to perform a continuous depolymerization reaction. At this point, a sweeping gas spray nozzle was installed in the residue treatment unit, and nitrogen gas (200°C), which was a sweeping gas, was sprayed at a supply rate of 0.5 cm/min in the counter-current to the inflow of the depolymerization residue. The depolymerization product was liquefied without installing a separation column unit. Then, the liquefied product was subjected to measurement using a GC/FID (Younglin Instruments) equipped with a capillary column (HP-5, 30 m $\times$ 0.32 mm $\times$ 1.0 $\mu$m, Crosslinked 5 % PH ME Siloxane). The results are shown in Table 4 and FIG. 5 below. $\alpha_1$, $\alpha_2$, and $\alpha_3$ in Table 4 below were calculated using Formulas 1 to 3 above.

[Table 4]

| Division | Catalyst content (wt%) | Yield (%) | | | | | | $\alpha_1$ | $\alpha_2$ | $\alpha_3$ |
| | | Liquid oil | Dimers+ | Toluene | EB | SM | α-MS | | | |
|---|---|---|---|---|---|---|---|---|---|---|
| Experimental Example 3-1 | 0 | 77.7 | 7.4 | 2.6 | 1.5 | 61.9 | 4.3 | 41.3 | 7.4 | 8.4 |
| Experimental Example 3-2 | 1 | 87.9 | 2.4 | 1.8 | 0.1 | 80.7 | 2.9 | 807.0 | 16.8 | 33.6 |
| Experimental Example 3-3 | 3 | 88.3 | 3.2 | 1.9 | 0.2 | 80.1 | 2.9 | 400.5 | 16.0 | 25.0 |
| Experimental Example 3-4 | 5 | 87.1 | 4.1 | 2.6 | 0.5 | 77.8 | 2.1 | 155.6 | 15.0 | 19.0 |
| SM: styrene monomer, EB: Ethylbenzene, α-MS: alphamethylstyrene | | | | | | | | | | |

As shown in Table 4 and FIG. 5, it was confirmed that in Experimental Example 3-1, in which no catalyst was added, the yield of styrene monomer was significantly low compared to those in Experimental Examples 3-2 to 3-4, in which a catalyst was added. In particular, in the case of Experimental Example 3-2, in which the catalyst content was added at 1 wt%, it was confirmed that the catalyst content showed excellent performance in terms of yield and by-product production. As the catalyst content increased to 3wt% and 5wt%, side reactions accelerated and the yield of styrene monomer tended to decrease.

**[Experimental Example 4: Measurement of yield of depolymerization product depending on catalyst type for depolymerization reaction]**

In Experimental Examples 4-1 to 4-10, depolymerization was performed in the same manner as in Experimental Example 1-1, but the conditions were changed to those shown in Table 5, and continuous depolymerization and depolymerization were performed in an Auger reactor and batch reactor, respectively. At this point, in Experimental Examples 4-7 to 4-10, 1 kg of waste polystyrene (PS) shredded to an average particle size of 0.5 cm and 50 g (5 wt%) of catalyst were mixed in a 3 L batch reactor. Next, the reactor was heated to 425°C, and the depolymerization reaction was performed at this temperature for 1 hour. The product decomposed by the depolymerization reaction was obtained by liquefying it in a condenser. The product was analyzed using GC/FID (Younglin Instruments) equipped with a capillary column (HP-5, 30 m $\times$ 0.32 mm $\times$ 1.0 $\mu$m, Crosslinked 5 % PH ME Siloxane). The results are shown in Table 6 below. $\alpha_1$, $\alpha_2$, and $\alpha_3$ in Table 6 below were calculated using Formulas 1 to 3 above.

[Table 5]

| Division | Depolymerization reactor | Catalyst type | Catalyst content (wt%) | Depolymerization Reaction temperature (° C) | Residence time (hr) |
|---|---|---|---|---|---|
| Experimental Example 4-1 | Auger reactor | $K_2CO_3$ | 5 | 425 | 1 |
| Experimental Example 4-2 | Auger reactor | $Na_2CO_3$ | 5 | 425 | 1 |
| Experimental Example 4-3 | Auger reactor | $BaCO_3$ | 5 | 425 | 1 |
| Experimental Example 4-4 | Auger reactor | $MgCO_3$ | 5 | 425 | 1 |
| Experimental Example 4-5 | Auger reactor | KOH | 5 | 425 | 1 |
| Experimental Example 4-6 | Auger reactor | $Mg(OH)_2$ | 5 | 425 | 1 |
| Experimental Example 4-7 | Auger reactor | $Ca(OH)_2$ | 5 | 425 | 1 |
| Experimental Example 4-8 | Batch reactor | $K_2CO_3$ | 5 | 425 | 1 |
| Experimental Example 4-9 | Batch reactor | $Na_2CO_3$ | 5 | 425 | 1 |
| Experimental Example 4-10 | Batch reactor | $BaCO_3$ | 5 | 425 | 1 |

(continued)

| Division | Depolymerization reactor | Catalyst type | Catalyst content (wt%) | Depolymerization Reaction temperature (° C) | Residence time (hr) |
|---|---|---|---|---|---|
| Experimental Example 4-11 | Batch reactor | KOH | 5 | 425 | 1 |
| Experimental Example 4-12 | Batch reactor | $Mg(OH)_2$ | 5 | 425 | 1 |
| Experimental Example 4-13 | Batch reactor | $Ca(OH)_2$ | 5 | 425 | 1 |

[Table 6]

| Division | Yield (%) | | | | | | $\alpha_1$ | $\alpha_2$ | $\alpha_3$ |
|---|---|---|---|---|---|---|---|---|---|
| | Liquid oil | Dimers+ | Toluene | EB | SM | $\alpha$-MS | | | |
| Experimental Example 4-1 | 81.1 | 3.5 | 2.6 | 0.4 | 72.8 | 1.8 | 182.0 | 15.2 | 20.8 |
| Experimental Example 4-2 | 77.2 | 3.2 | 2.6 | 2.3 | 66.7 | 2.4 | 29.0 | 9.1 | 20.8 |
| Experimental Example 4-3 | 71.1 | 1.5 | 3.3 | 2.9 | 59.5 | 3.9 | 20.5 | 5.9 | 39.7 |
| Experimental Example 4-4 | 76.7 | 1.2 | 4.6 | 5.7 | 59.1 | 6.1 | 10.4 | 3.6 | 49.3 |
| Experimental Example 4-5 | 49.7 | 1.5 | 1.7 | 1.2 | 43.8 | 1.5 | 36.5 | 10.0 | 29.2 |
| Experimental Example 4-6 | 61.6 | 6.5 | 4.1 | 1.7 | 43.4 | 5.9 | 25.5 | 3.7 | 6.7 |
| Experimental Example 4-7 | 72.3 | 4.1 | 8.4 | 6 | 44.2 | 9.6 | 7.4 | 1.8 | 10.8 |
| Experimental Example 4-8 | 61.6 | 6.5 | 4.1 | 1.7 | 43.4 | 5.9 | 25.5 | 3.7 | 6.7 |
| Experimental Example 4-9 | 73.6 | 6.2 | 5.2 | 2.5 | 51.7 | 8 | 20.7 | 3.3 | 8.3 |
| Experimental Example 4-10 | 73.2 | 4.3 | 6.6 | 2.4 | 51.3 | 8.6 | 21.4 | 2.9 | 11.9 |
| Experimental Example 4-11 | 63.2 | 1.8 | 2.3 | 1.1 | 55.9 | 2.1 | 50.8 | 10.2 | 31.1 |
| Experimental Example 4-12 | 69.0 | 2.2 | 2.5 | 2.9 | 58.9 | 2.5 | 20.3 | 7.5 | 26.8 |
| Experimental Example 4-13 | 69.4 | 2.1 | 4.3 | 3.4 | 54.2 | 5.4 | 15.9 | 4.1 | 25.8 |
| SM: styrene monomer, EB: Ethylbenzene, $\alpha$-MS: alphamethylstyrene | | | | | | | | | |

As shown in Table 6, in the case of carbonate catalyst as in Experimental Examples 4-1 to 4-4, the yield of styrene monomer was found to be high compared to those in Experimental Examples 4-5 and 4-6 using a hydroxide catalyst. Among the carbonate catalysts of Experimental Examples 4-1 to 4-4, it was confirmed that the yield of styrene monomer in Experimental Examples 4-1 and 4-2 using an alkali metal carbonate catalyst was high. In particular, in the case of Experimental Example 4-1 using a potassium carbonate ($K_2CO_3$) catalyst, the yield and $\alpha_1$ and $\alpha_2$ values of styrene monomer were found to increase significantly in the continuous reaction compared to the batch reactor. This was not limited by theory but could be assumed to be due to the effect of the mass transfer limitation of solid (catalyst) - liquid (reactant) - gas (product). In the case of potassium carbonate, which has a relatively high decomposition temperature under the depolymerization temperature, it was assumed that the mixing of reactants and catalysts could be maintained uniformly in a continuous reaction. Meanwhile, in the case of solid catalysts in batch reactions, it was assumed that the contact between the catalyst and reactants was uniformly reduced due to the density difference.

**[Experimental Example 5: Measurement of recovery rate of depolymerization product depending on temperature of residue treatment unit]**

In Experimental Example 5, to measure the recovery rate of the depolymerization product depending on the temperature of the residue treatment unit, depolymerization was performed in the same manner as in Experimental Example 1-4, but the process conditions after the use of the Auger reactor were changed to the conditions shown in Table 7 to conduct continuous depolymerization. At this point, a sweeping gas spray nozzle was installed in the residue treatment unit, and nitrogen gas (200°C), which was a sweeping gas, was sprayed at a supply rate of 10 cm/min in the counter-current to the inflow of the depolymerization residue. The depolymerization product was liquefied using a separation column unit filled with a filler material (borosilicate, Raschig ring, 6 mm × 6 mm) at a filling rate of 75%. Then, the liquefied product was subjected to measurement using a GC/FID (Younglin Instruments) equipped with a capillary column (HP-5, 30 m × 0.32

mm × 1.0 μm, Crosslinked 5 % PH ME Siloxane). The results are shown in Table 7 below.

[Table 7]

| Division | Temperature of residue treatment unit (° C) | Yield (%) | | Residue |
|---|---|---|---|---|
| | | Total oil | SM | |
| Experimental Example 5-1 | 25 | 59.62 | 56.5 | liquid |
| Experimental Example 5-2 | 200 | 87.1 | 77.8 | liquid |
| Experimental Example 5-3 | 400 | 58.36 | 49.8 | solid |
| SM: styrene monomer | | | | |

As shown in Table 7, in the case of Experimental Example 5-2 in which the residue treatment unit was heated to 200°C, the yield of recovered styrene monomer was found to be high compared to those in Experimental Examples 5-1 and 5-3 in which the residue treatment unit was not heated or was heated to 400°C. When the temperature of the residue treatment unit was not heated as in Experimental Example 5-1, the high-temperature gas product was instantaneously cooled and moved to the residue treatment unit, thereby reducing the yield of styrene monomer. In addition, when the residue treatment unit was heated to a high temperature of 400°C as in Experimental Example 5-3, it was confirmed that radical polymerization of styrene monomer progressed rapidly, and the yield rapidly decreased.

**[Experimental Example 6: Measurement of recovery rate of depolymerization product depending on supply of sweeping gas and filling of separation column unit]**

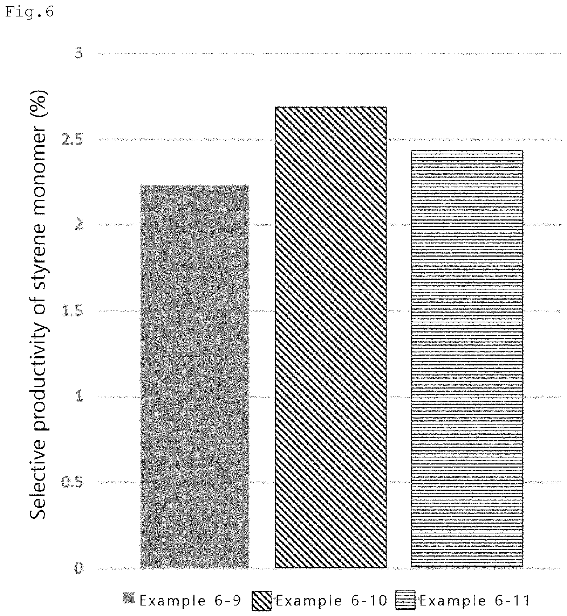

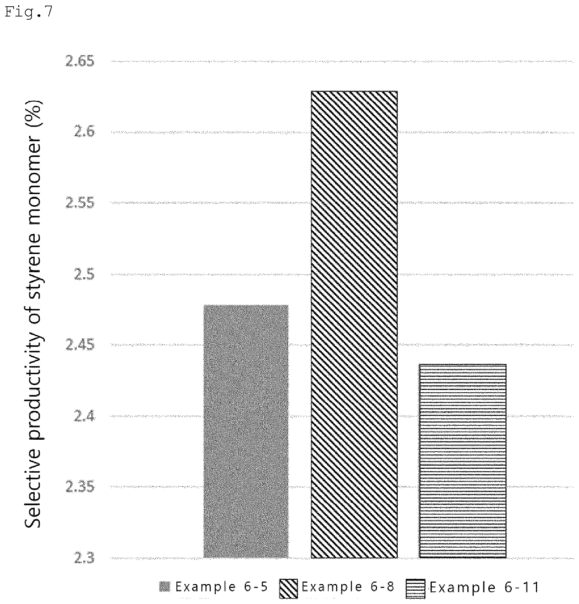

In Experimental Example 6, to measure the recovery rate of depolymerization product depending on the supply rate of the sweeping gas and the filling of the separation column unit, depolymerization was performed in the same manner as in Experimental Example 1-4, but the process conditions after the use of the Auger reactor were changed to the conditions shown in Tables 8 and 9 to conduct continuous depolymerization. At this point, a sweeping gas spray nozzle was installed in the residue treatment unit, and nitrogen gas (200°C), which was a sweeping gas, was sprayed in the counter-current to the inflow of the depolymerization residue. The depolymerization product was liquefied using a separation column unit filled with a filler material (borosilicate, Raschig ring, 6 mm × 6 mm) and a separation column unit without a filler material. Then, the liquefied product was subjected to measurement using a GC/FID (Younglin Instruments) equipped with a capillary column (HP-5, 30 m × 0.32 mm × 1.0 μm, Crosslinked 5 % PH ME Siloxane). The results are shown in Tables 8 and 9 and FIGs. 6 and 7. At this point, the β selectivity and selective productivity of styrene monomer were calculated using Formulas 4 and 5 below.

β Selectivity (%) = [(Y styrene/Y dimer)/(X styrene/X dimer)]= (composition discharged from separation column/stock solution composition [Formula 4]                    [Formula 4]

Selective productivity of styrene monomer (g/h) = Purity of styrene monomer × Productivity of styrene monomer [Formula 5]                    [Formula 5]

In the above formula, the productivity of styrene monomer refers to the amount of styrene monomer produced in the reactor per unit time.

[Table 8]

| Division | Presence of Seperation column unit | Supply rate of sweeping gas (cm/min ) | Filling rate of filler (%) | Division | Yield (%) or selectivity (%) | | | | | |
|---|---|---|---|---|---|---|---|---|---|---|
| | | | | | Liquid oil | Dimers + | Toluene | EB | styrene | a-MS |
| Experimental Example 6-1 | nonexistence | 0 | - | Yield | 70.9 | 4.5 | 1.5 | 1.1 | 60.6 | 3.2 |
| | | | | Selectivity | - | 6.3 | 2.1 | 1.6 | 85.5 | 4.5 |

(continued)

| Division | Presence of Seperation column unit | Supply rate of sweepin g gas (cm/min ) | Fillin g rate of filler (%) | Division | Yield (%) or selectivity (%) | | | | | |
|---|---|---|---|---|---|---|---|---|---|---|
| | | | | | Liqui d oil | Dimers + | Toluen e | EB | styren e | a-MS |
| Experiment al Example 6-2 | nonexisten ce | 0.5 | - | Yield | 87.1 | 4.1 | 2.6 | 0. 5 | 77.8 | 2. 1 |
| | | | | Selectivit y | - | 4.7 | 3.0 | 0. 6 | 89.3 | 2. 4 |

[Table 9]

| Division | Presence of Separation column unit | Supply rate of sweeping gas (cm/min) | Filling rate of filler (%) | Selectivity (%) | | | | | |
|---|---|---|---|---|---|---|---|---|---|
| | | | | β | Dimers+ | Toluene | EB | styrene | a-MS |
| Experimental Example 6-2 | nonexistence | 0.5 | - | 1 | 4.7 | 3.0 | 0.6 | 89.3 | 2.4 |
| Experimental Example 6-3 | existence | 0.5 | - | 52 | 0.3 | 4.1 | 0.7 | 92.3 | 2.6 |
| Experimental Example 6-4 | existence | 2.5 | - | 30 | 0.6 | 4 | 0.6 | 92.8 | 2 |
| Experimental Example 6-5 | existence | 10.0 | - | 20 | 0.7 | 5.4 | 0.6 | 89.3 | 4 |
| Experimental Example 6-6 | existence | 0.5 | 25 | 59 | 0.3 | 3.3 | 0.7 | 94.6 | 1.1 |
| Experimental Example 6-7 | existence | 2.5 | 25 | 50 | 0.4 | 4.2 | 0.6 | 92.7 | 2.1 |
| Experimental Example 6-8 | existence | 10.0 | 25 | 43 | 0.4 | 5.2 | 0.6 | 89.4 | 4.4 |
| Experimental Example 6-9 | existence | 0.5 | 50 | 77 | 0.2 | 3.6 | 0.7 | 94.6 | 0.9 |
| Experimental Example 6-10 | existence | 2.5 | 50 | 73 | 0.2 | 4 | 0.6 | 92.8 | 2.4 |
| Experimental Example 6-11 | existence | 10.0 | 50 | 56 | 0.3 | 4.4 | 0.6 | 92.4 | 2.3 |

As shown in Table 8, when nitrogen gas, which was a sweeping gas, was supplied to the residue treatment unit equipped with a sweeping gas spray nozzle as in Experimental Example 6-2, it was confirmed that both the yield and selectivity of styrene monomer increased compared to Experimental Example 6-1 in which sweeping gas was not supplied.

In addition, as shown in Table 9, it was confirmed that under the same conditions, the higher the supply rate of the sweeping gas, the lower the selectivity of recovered styrene monomer, whereas the lower the supply rate of sweeping gas, the lower the yield of recovered styrene monomer. In addition, when comparing Experimental Examples 6-2 and 6-3, it was confirmed that the production of dimer+ was significantly reduced in the case where the separation column unit existed compared to the case in which the separation column unit did not exist. It was also found that the higher the filling ratio inside the separation column unit, the higher the selectivity of recovered styrene monomer.

Accordingly, considering the selective productivity of styrene monomer as shown in FIGs. 6 and 7, selectivity and yield were inversely proportional to each other, so selectivity and yield were required to be appropriately adjusted considering the situation.

Although the preferred examples of the present disclosure have been described above, the present disclosure is not limited thereto. The present disclosure can be implemented with various modifications within the scope of the claims, the detailed description of the present disclosure, and the accompanying drawings. It is natural that the various modifications also fall within the scope of the present disclosure.

**Claims**

1.  A method of continuously recovering a styrene monomer from waste polystyrene by continuous depolymerization of the waste polystyrene, the method comprising:

    obtaining a styrene monomer-containing product by continuously introducing waste polystyrene and a potassium carbonate ($K_2CO_3$) catalyst into a depolymerization reactor and depolymerizing the mixture,
    wherein the yield of the styrene monomer (SM) by the depolymerization is 70% or more and satisfies one or more of Formula 1 or 2 below.

    $$\text{(styrene yield)}/\text{(ethylbenzene yield)} \geq 90 \quad [\text{Formula 1}]$$

    (styrene yield) / (toluene yield + ethylbenzene yield + $\alpha$-methylstyrene yield) $\geq$ 12 [Formula 2]     [Formula 2]

2.  The method of claim 1, wherein the potassium carbonate ($K_2CO_3$) catalyst is introduced in an amount of 0.1 to 10 parts by weight based on 100 parts by weight of the waste polystyrene.

3.  The method of claim 1, comprising:

    (a) obtaining a styrene monomer-containing depolymerization product by continuously introducing waste polystyrene and a potassium carbonate ($K_2CO_3$) catalyst into a depolymerization reactor made of one or two or more stages and depolymerizing the mixture;
    (b) discharging a gaseous depolymerization product out of the depolymerization products to a depolymerization gas discharge unit using a sweeping gas, and discharging a depolymerization residue other than the gaseous depolymerization product to a residue treatment unit; and
    (c) obtaining a styrene monomer by collecting the discharged gaseous depolymerization product.

4.  The method of claim 3, wherein the step (b) involves supplying the sweeping gas in a counter-current to the discharge flow of the depolymerization residue to separate the styrene monomer from the depolymerization residue being discharged to a residue treatment unit and discharge the styrene monomer to the depolymerization gas discharge unit.

5.  The method of claim 3, wherein, in the step (b), when the depolymerization reactor is made of two or more stages, each of depolymerization gas discharge units, between each stage, is connected in communication with adjacent units, allowing the gaseous depolymerization product among the depolymerization products generated in each stage of the depolymerization reactor to be discharged outside the depolymerization reactor and allowing the depolymerization residue other than the gaseous depolymerization product to be moved to the rear stage,
    the sweeping gas is further supplied in a counter-current to the flow of the depolymerization residue moving from stage to stage, thereby the styrene monomer remaining in the depolymerization residue moving between each of the stages is discharged from each of the depolymerization gas discharge units.

6.  The method of any claim 3 or 5, wherein the step (c) further comprises reducing the content of dimer+ in the gaseous depolymerization product discharged from the depolymerization gas discharge unit.

7.  The method of claim 6, wherein reducing the content of dimer+ is performed in a separation column unit installed in the depolymerization gas discharge unit.

8.  The method of any claim 3 or 5, wherein the sweeping gas is heated and supplied.

9.  The method of claim 3, wherein the residence time of waste polystyrene in the depolymerization reactor is in a range of 15 minutes to 2 hours.

**10.** The method of claim 3, wherein the recovering of the potassium carbonate ($K_2CO_3$) catalyst present in the remaining residue is further comprised by bringing the remaining residue into contact with water after the styrene monomer has been discharged from the residue treatment unit.

EP 4 512 792 A1

Fig. 1

Fig. 2

18

Fig. 3

Fig.4

Fig.5

Fig.6

Fig.7

## INTERNATIONAL SEARCH REPORT

| International application No. |
|---|
| **PCT/KR2023/005183** |

**A. CLASSIFICATION OF SUBJECT MATTER**

**C07C 4/22**(2006.01)i; **C07C 15/46**(2006.01)i; **C08J 11/10**(2006.01)i

According to International Patent Classification (IPC) or to both national classification and IPC

**B. FIELDS SEARCHED**

Minimum documentation searched (classification system followed by classification symbols)

C07C 4/22(2006.01); B01D 3/00(2006.01); C07C 15/46(2006.01); C08G 63/78(2006.01); C08J 11/02(2006.01); C08J 11/12(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), Google & keywords: 폐 폴리스티렌(waste polystyrene), 해중합(depolymerization), 탄산칼륨 (potassium carbonate, K2CO3), 연속(continuous), 회수(recovery)

**C. DOCUMENTS CONSIDERED TO BE RELEVANT**

| Category* | Citation of document, with indication, where appropriate, of the relevant passages | Relevant to claim No. |
|---|---|---|
| A | KR 10-2003-0081717 A (KOREA RESEARCH INSTITUTE OF CHEMICAL TECHNOLOGY) 22 October 2003 (2003-10-22)<br>See paragraphs [0029] and [0030]; claims 1-3; and figure 1. | 1-10 |
| A | JP 2014-070132 A (TEIJIN LTD.) 21 April 2014 (2014-04-21)<br>See claims 1-6; and paragraph [0016]. | 1-10 |
| A | WO 2021-053074 A1 (INEOS STYROLUTION GROUP GMBH) 25 March 2021 (2021-03-25)<br>See claims 1-15. | 1-10 |
| A | KR 10-2021-0113263 A (INEOS STYROLUTION GROUP GMBH) 15 September 2021 (2021-09-15)<br>See claims 1-17. | 1-10 |
| A | KR 10-1838815 B1 (SOGANG UNIVERSITY RESEARCH & BUSINESS DEVELOPMENT FOUNDATION) 14 March 2018 (2018-03-14)<br>See entire document. | 1-10 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **28 July 2023** | **31 July 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT
Information on patent family members

| International application No. |
| --- |
| **PCT/KR2023/005183** |

| Patent document cited in search report | | | Publication date (day/month/year) | Patent family member(s) | | | Publication date (day/month/year) |
| --- | --- | --- | --- | --- | --- | --- | --- |
| KR | 10-2003-0081717 | A | 22 October 2003 | KR | 10-0468047 | B1 | 24 January 2005 |
| JP | 2014-070132 | A | 21 April 2014 | None | | | |
| WO | 2021-053074 | A1 | 25 March 2021 | EP | 4031610 | A1 | 27 July 2022 |
| | | | | KR | 10-2022-0065005 | A | 19 May 2022 |
| | | | | US | 2022-0324777 | A1 | 13 October 2022 |
| KR | 10-2021-0113263 | A | 15 September 2021 | CN | 113544108 | A | 22 October 2021 |
| | | | | EP | 3908563 | A1 | 17 November 2021 |
| | | | | US | 2022-0081372 | A1 | 17 March 2022 |
| | | | | WO | 2020-144165 | A1 | 16 July 2020 |
| KR | 10-1838815 | B1 | 14 March 2018 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001294708 A
- KR 200187093
- KR 20030081717

**Non-patent literature cited in the description**

- *Ind. Eng. Res.*, 1995, vol. 34 (12), 4519